# EUROPÄISCHE PATENTANMELDUNG

(11) **EP 4 484 990 A1**
(43) Veröffentlichungstag der Anmeldung: **01.01.2025**
(21) Anmeldenummer: 23182021.8
(22) Anmeldetag: 28.06.2023
(51) Int. Cl.: G01R 33/36, G01R 33/565, G01R 33/567

(54) **MAGNETRESONANZSYSTEM MIT INTEGRIERTER PILOTTONVORRICHTUNG UND VERFAHREN ZUM BETRIEB**

(71) Anmelder: Siemens Healthineers AG, 91301 Forchheim (DE)
(72) Erfinder: Die Erfindernennung liegt noch nicht vor
(74) Vertreter: Siemens Healthineers Patent Attorneys

(57) **Zusammenfassung**

Die Erfindung betrifft ein Magnetresonanzsystem mit einer Pilottonvorrichtung sowie ein Verfahren zum Betrieb des Magnetresonanzsystems. Das Magnetresonanzsystem weist einen Empfänger auf, der ausgebildet ist, ein Pilottonsignal und ein Magnetresonanzsignal gleichzeitig zu empfangen und auszuwerten. Der Empfänger weist weiterhin ein frequenzabhängiges Dämpfungsglied auf, das ausgebildet ist, ein empfangenes Pilottonsignal relativ zu einem empfangenen Magnetresonanzsignal zu dämpfen.

## Beschreibung

Unabhängig vom grammatikalischen Geschlecht eines bestimmten Begriffes sind Personen mit männlicher, weiblicher oder anderer Geschlechteridentität mit umfasst.

Die Erfindung betrifft ein Magnetresonanzsystem mit einer Pilottonvorrichtung. Das Magnetresonanzsystem weist einen Empfänger auf, der ausgebildet ist, ein Pilottonsignal und ein Magnetresonanzsignal gleichzeitig zu empfangen und auszuwerten.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Magnetresonanzsignal wird dann ausgewertet und eine zwei- oder dreidimensionale bildgebende Darstellung des Untersuchungsobjektes bereitgestellt.

Die Magnetresonanzsignale sind sehr schwach. Um ein ausreichend hohes Signal-zu-Rausch-Verhältnis zu erzielen, ist daher das Signal über eine lange Zeit zu erfassen, in einer oder in wiederholten Messungen. Die Erfassung der Magnetresonanzsignale ist dabei langsam im Vergleich zu unvermeidbaren Bewegungen des Patienten wie Herzschlag oder Atembewegung.

Die Bewegungen verursachen dabei Artefakte in den erzeugten Abbildungen.

Eine Möglichkeit, die bewegten Organe abzubilden besteht dennoch, wenn eine kurze Bilderfassung synchronisiert zu der Bewegung wiederholt ausgeführt wird und über die erfassten Daten mittelt.

Eine Synchronisierung kann mit dedizierten Sensoren wie beispielsweise Atemgurt oder EKG-Elektroden erfolgen.

Um diese zusätzlichen Sensoren zu vermeiden, ist es auch schon aus der Druckschrift US 2015/0320342 A1 bekannt, ein kontinuierliches, monofrequentes Magnetwechselfeld von einer kleinen Leiterschleife ausgehend, zumindest teilweise durch den Körper des Patienten hindurch in die einzelnen Elemente einer Magnetresonanz-Lokalspule zu koppeln.

Da die meisten biologischen Gewebe fast vollständig transparent für Magnetfelder sind, durchdringt das erzeugte Magnetfeld den Körper des Patienten fast unverändert. Die meisten Gewebe sind jedoch (schwach) leitend und daher induziert das kontinuierliche Wellenmagnetfeld Wirbelströme. Diese Wirbelströme erzeugen dann wiederum ein Magnetfeld, das das Anregungsfeld überlagert, was zu Modulationen im empfangenen Magnetfeld in der Empfangsspule führt.

Durch Auswertung dieses Signals kann auf eine Bewegungsphase des Herzens oder der Atmung geschlossen werden.

Bei Verwendung eines Pilottonsignals kann es aber vorkommen, dass die empfangenen Signale und damit auch die durch die Bewegung verursachten Amplitudenschwankungen sehr schwach und kaum auszuwerten sind. Dies ist insbesondere der Fall, wenn das Pilottonsignal und das Magnetresonanzsignal gleichzeitig mit einem gemeinsamen Empfänger empfangen und ausgewertet werden, da der nutzbare Amplitudendynamikbereich des Empfangssystems nicht durch das Pilottonsignal eingeschränkt werden darf, wodurch der maximal erzielbare Pilottonsignal-Empfangspegel begrenzt wird. Auch kann es vorkommen, dass der Anteil des Magnetwechselfeldes, welches durch den Herzschlag bewegtes Gewebe durchdringt, zu gering ist und das demodulierte Empfangssignal auch in diesem Fall ein zu geringes Signal-zu-Rauschverhältnis aufweist.

Es ist daher eine Aufgabe der vorliegenden Erfindung, das Erfassen von Bewegungen des Patienten besser und zuverlässiger zu machen.

Die Aufgabe wird durch eine erfindungsgemäße Magnetresonanzsystem nach Anspruch 1 gelöst.

Das erfindungsgemäße Magnetresonanzsystem weist eine Pilottonvorrichtung auf. Die Pilottonvorrichtung koppelt ein hochfrequentes magnetisches Wechselfeld aus, das nachfolgend auch als Pilotton bezeichnet wird. Das Wechselfeld wechselwirkt mit einem Körper eines Patienten durch induzierte Ströme und Suszeptibilitätssprüngen an Organgrenzen. Diese Wechselwirkung ändert sich mit Bewegungen des Körpers und führt zu einer Modulation des Pilottons in ein Pilottonsignal, das ein Empfänger der Pilottonvorrichtung aufnimmt und so eine Bewegung des bzw. im Körper des Patienten erfassen kann.

Das erfindungsgemäße Magnetresonanzsystem weist einen Magnetresonanztomographen zum Empfang eines Magnetresonanzsignals zur Abbildung eines Patienten auf. In dem erfindungsgemäßen Magnetresonanzsystem wird ein Empfänger des Magnetresonanztomographen auch dazu genutzt, gleichzeitig mit dem Magnetresonanzsignal das Pilottonsignal zu empfangen. So lassen sich vorhandene Signalverarbeitungsressourcen doppelt nutzen. Dabei muss die Pilottonvorrichtung ausgebildet sein, einen Pilotton in einem Frequenzbereich zu erzeugen, der auch von dem Empfänger erfasst wird. Mit anderen Worten, der Frequenzbereich des Pilottons ist nahe der Larmorfrequenz, die von den zu erfassenden Kernspins und einem statischen Magnetfeld B0 des Magnetresonanztomographen vorbestimmt ist. Der Pilotton weist eine Frequenz auf, die geringfügig von der Larmorfrequenz abweicht, beispielsweise um weniger als 5%, 2 % oder 1% und nicht in das vom MR-Signal belegte Frequenzband fällt.

Vorzugsweise werden für das Pilottonsignal und Magnetresonanzsignal auch weitere Ressourcen gemeinsam genutzt. Insbesondere ist es denkbar, dass das das Magnetresonanzsystem einen gemeinsamen Empfangspfad für das Pilottonsignal und das Magnetresonanzsignal einen gemeinsamen Signalweg bzw. Empfangspfad bis zum Empfänger mit Antennenspulen und/oder Vorverstärker bzw. Low-Noise-Verstärker (LNA) aufweist.

Das erfindungsgemäße Magnetresonanzsystems weist weiterhin ein frequenzabhängiges Dämpfungsglied auf, das ausgebildet ist, ein empfangenes Pilottonsignal relativ zu einem empfangenen Magnetresonanzsignal zu dämpfen. Mit anderen Worten, das frequenzabhängige Dämpfungsglied weist eine frequenzabhängige Kennlinie auf, die keine oder nur eine geringfügige Dämpfung für das Magnetresonanzsignal verursacht und eine vorbestimmte stärkere Dämpfung für das Pilottonsignal. Das Verhältnis der Dämpfung für das Magnetresonanzsignal und das Pilottonsignal nimmt dabei einen vorbestimmten Wert an.

Das Dämpfungsglied ist dabei im Signalweg vor einem A/D-Wandler des Empfängers und insbesondere zwischen dem Vorverstärker und dem A/D-Wandler angeordnet und vorzugsweise als analoge Schaltung realisiert. Denkbar ist es auch, dass das Dämpfungsglied integraler Bestandteil des Vorverstärkers ist, beispielsweise als frequenzabhängige Gegenkopplung.

Hierbei wird zwischen den Begriffen Pilotton und Pilottonsignal unterschieden. Mit Pilotton wird das Signal bezeichnet, dass von der Pilottonvorrichtung erzeugt und in den Körper des Patienten abgestrahlt wird. Mit Pilottonsignal wird hingegen ein aus dem Pilotton resultierendes Signal bezeichnet, dass im Empfänger ausgewertet wird. Das Pilottonsignal weist zum einen eine erwünschte Schwankung auf, die durch Bewegungen des bzw. im Körper des Patienten eine Amplitudenschwankung bzw. Modulation des sich ausbreitenden und von der Lokalspule empfangenen Pilottons hervorrufen. Zum anderen kann das Pilottonsignal einer weiteren Vorbehandlung unterliegen, beispielsweise eine Filterung durch die Frequenzcharakteristik der Lokalspule oder eines Bandpassfilters, Vorverstärkung durch einen LNA oder eine Frequenzumsetzung auf eine Zwischenfrequenz. Diese betreffen jedoch von der Lokalspule aufgenommenen Magnetresonanzsignale und den modulierten Pilotton im Wesentlichen auf gleiche Weise, sodass z.B. Frequenzabstand und Amplitudenverhältnis ganz bzw. im Wesentlichen unverändert bleiben. Ausführungen bezüglich der Larmorfrequenz des Magnetresonanzsignals betreffen daher für das Pilottonsignal gegebenenfalls entsprechend umgesetzte Frequenzen eines Zwischenfrequenzsignals.

In heutigen MR-Empfangssystemen wird der nutzbare Amplitudendynamikbereich durch die Eigenschaften der Analog-Digitalwandler (ADC) bestimmt. Durch das Einfügen einer Dämpfung vor dem ADC kann der verfügbare Amplitudendynamikbereich des Empfangssystems auf Kosten einer leichten Erhöhung der Rauschzahl erhöht werden. Da die Rauschzahl des Empfängers und damit das Grundrauschen deutlich geringer ansteigen als die eingesetzte Dämpfung, kann auf vorteilhafte Weise das SNR des empfangenen Pilottonsignals somit vergrößert werden, wenn der Pegel des ausgesendeten Pilottons und damit das Pilottonsignal in gleichem Maße angehoben wird, wie die Empfängerverstärkung mittels des Dämpfungsgliedes reduziert wird. Der nutzbare Amplitudendynamikbereich des MR-Empfangspfades bleibt hiervon unberührt.

Weitere vorteilhafte Ausführungsformen sind zu den Unteransprüchen angegeben.

In einer denkbaren Ausführungsform des erfindungsgemäßen Magnetresonanzsystems ist die Dämpfung des empfangenen Pilottonsignals gegenüber dem empfangenen Magnetresonanzsignal durch das Dämpfungsglied größer als 3 dB, 6dB oder 9 dB. Vorzugsweise ist die Dämpfung aber auch nicht größer als 12 dB, 18 dB oder 24 dB.

Um den Empfang des Magnetresonanzsignals nicht zu stören, muss das empfangene Pilottonsignal einen deutlich geringeren Pegel aufweisen, insbesondere am Eingang eines gemeinsamen A/D-Wandlers. Um einen Signal-zu-Rausch-Abstand (SNR) des Pilotton-Signals zu erhöhen, wird gemäß der Erfindung ein Pegel des ausgesendeten Pilottons um einen vergleichbaren Pegel angehoben, um den es dann durch das Dämpfungsglied wieder reduziert wird. Dadurch kann auf vorteilhafte Weise trotz des gleichen Pegelabstandes zwischen Magnetresonanzsignal und Pilottonsignal am Eingang des A/D-Wandlers nach dem Dämpfungsglied das SNR des Pilottonsignals um einen Wert angehoben werden, der in etwa der Dämpfung bzw. der Erhöhung des gesendeten Pilottons entspricht. Nach oben ist der Wert durch die Begrenzung des auszusendenden Pilottons durch den begrenzten Amplitudendynamikbereich der vorgelagerten Signalaufbereitung wie Vorverstärker und/oder Mischer, SAR, regulatorische Begrenzungen und/oder Störungen anderer Einheiten durch den Pilotton beschränkt. Grundsätzlich ist es aber auch denkbar, das Dämpfungsglied vor der Signalaufbereitung im Empfangspfad anzuordnen, allerdings wird dann auch ein Rauschanteil z.B. durch thermisches Rauschen von dem Vorverstärker mit verstärkt.

In einer möglichen Ausführungsform des erfindungsgemäßen Magnetresonanzsystems ist das Magnetresonanzsystem ausgebildet, eine Dämpfung des Dämpfungsgliedes durch einen erhöhten Sendepegel der Pilottonvorrichtung für den Pilotton zu kompensieren. Wenn das Dämpfungsglied den Pilottonsignalpegel beispielsweise um 6 dB reduziert, so ist entsprechend der von der Pilottonvorrichtung ausgesendete Pilottonpegel um den Faktor 2 bzw. 6dB höher. Als Vergleichspegel kann hier ein Pilottonsignal eines Systems aus dem Stand der Technik dienen oder auch ein Pegel eines maximal auszuwertenden Magnetresonanzsignals. Mit anderen Worten, durch das Dämpfungsglied und die Anhebung des Pegels des Pilottons wird erreicht, dass am Eingang des A/D-Wandlers der Pegel des Pilottonsignals im Wesentlichen unverändert ist im Vergleich zu einem Magnetresonanztomographen aus dem Stand der Technik ohne das erfindungsgemäße Dämpfungsglied. Insbesondere wird der Pegelabstand zwischen dem Magnetresonanzsignal und dem Pilottonsignal nach dem Dämpfungsglied auf dem gleichen Wert wie bei einem Magnetresonanzsystem aus dem Stand der Technik gehalten.

Wie bereits zuvor beschrieben, wird durch den erhöhten Sendepegel für den Pilotton ein besseres SNR für das empfangene Pilottonsignal erzielt.

In einer denkbaren Ausführungsform des erfindungsgemäßen Magnetresonanzsystem nach einem der vorhergehenden Ansprüche weist das Dämpfungsglied einen Notch-Filter (Kerb-Filter) auf. Mit Notch-filter wird ein Filter bezeichnet, der beim Durchgang lediglich ein schmalbandiges Signal im Pegel reduziert und andere Frequenzbereiche im Wesentlichen nicht verändert, also beispielsweise eine Welligkeit von weniger als 0,5 dB oder 0,1 dB aufweist. Als schmalbandig wird dabei ein Signal angesehen, das eine Bandbreite in der Größenordnung einer Bandbreite des Pilottonsignals aufweist, also weniger als 500 Hz, 100 Hz, 50 Hz oder 10 Hz. Denkbar ist auch eine Dimensionierung, bei der mehrere Pilottonsignale, die spektral eng benachbart angeordnet liegen, mit einem gemeinsamen Filter gedämpft werden.

Auf vorteilhafte Weise dämpft ein Notch-Filter lediglich das Pilottonsignal und lässt das Magnetresonanzsignal im Wesentlichen unverändert.

In einer möglichen Ausführungsform des erfindungsgemäßen Magnetresonanzsystems weist das Dämpfungsglied einen Quarz-Resonator auf.

Quarz-Resonatoren weisen eine vorteilhafte Frequenzstabilität auf.

In einer denkbaren Ausführungsform des erfindungsgemäßen Magnetresonanztomographen weist das Dämpfungsglied eine Mehrzahl an gekoppelten Resonatoren auf. Als Resonatoren werden dabei Filterelemente bezeichnet, die eine Resonanzfrequenz, also eine bevorzugte Durchlass- oder Dämpfungsfrequenz aufweisen. Eine Kopplung ist dabei eine Wechselwirkung zwischen den Resonatoren, die in beiden Richtungen zwischen den beteiligten Resonatoren erfolgt. Eine Kopplung kann beispielsweise induktiv, kapazitiv und/oder resistiv erfolgen.

Auf vorteilhafte Weise kann durch wechselwirkende Resonatoren die Charakteristik eine Dämpfungsgliedes oder Bandpassfilters beeinflusst werden, insbesondere die Bandbreite eines Durchlassbereichs. Hierdurch kann die Bandbreite und die Dämpfung derart gewählt werden, dass sich ein tragbarer Kompromiss aus erzielter Dämpfung auf der PR-Signalfrequenz, geringer Beeinflussung des MR-Signals und möglichst kurzer Einschwingzeit ergibt.

Denkbar sind als Dämpfungsglied auch Hochpass- bzw. Tiefpassfilter. Dabei liegt die Flanke des Filters zwischen der Frequenz des Pilottons und den Frequenzen des Magnetresonanzsignals, insbesondere der Larmorfrequenz des Magnetresonanzsystems, die durch das magnetische Moment der zu erfassenden Kernspins und das statische Magnetfeld B0 des Magnetresonanzsystems vorgegeben ist. Liegt die Frequenz des Pilottons oberhalb der Larmorfrequenz, wird ein Tiefpassfilter eingesetzt, um das Pilottonsignal zu dämpfen, entsprechend bei einer Frequenz des Pilottons unterhalb der Larmorfrequenz ein Hochpassfilter. Dabei kann alternativ oder zusätzlich eine Frequenzselektivität einer resonanten Antennenspule genutzt werden, um spektral weiterabliegende Pilottonsignale zu dämpfen.

Möglich wäre aber auch eine Kombination eines Hochpass- und eines Tiefpassfilters als Dämpfungsglied bzw. als Notch-Filter.

Auf vorteilhafte Weise lassen sich mit Hoch- und Tiefpassfiltern besonders schnelle Einschwingverhalten und/oder geringe Welligkeit im Durchlassbereich erzielen.

In einer möglichen Ausführungsform des erfindungsgemäßen Magnetresonanzsystems weist das Magnetresonanzsystem eine Mehrzahl an Empfangskanälen auf. Die Empfangskanäle können beispielsweise zum parallelen Empfang von Magnetresonanzsignalen einer Lokalspulenmatrix vorgesehen sein. Die Mehrzahl an Empfangskanälen weist jeweils einen Empfänger und ein erfindungsgemäßes frequenzabhängiges Dämpfungsglied auf. Das Dämpfungsglied kann dabei Teil eines Empfängers sein, aber beispielsweise auch Teil eines Vorverstärkers bzw. LNA oder zwischen LNA und Empfänger angeordnet sein. Dabei ist es denkbar, dass die Dämpfungsglieder jeweils unterschiedliche Dämpfungswerte aufweisen. Der Dämpfungswert hängt dabei vorzugsweise von einem zu erwartenden Empfangspegel des Pilottons in einem anwendungsgemäßen Betrieb ab. Der jeweilige Dämpfungswert ist dabei so ausgelegt, dass das Pilottonsignal in allen Empfängern nach dem Dämpfungsglied im wesentlichen gleichen Pegel aufweist.

Beispielsweise wird der Empfangspegel von einem Abstand und einer geometrischen Anordnung einer angeschlossenen Antennenspule zu einer Sendeantenne für den Pilotton beeinflusst.

Es ist auch denkbar, dass das bzw. die Dämpfungsglieder eine von einer Steuerung des Magnetresonanzsystems einstellbare Dämpfung aufweisen, sodass die Dämpfung von der Steuerung so eingestellt werden kann, dass am Empfänger das Pilottonsignal auf einen vorbestimmten Pegel reduziert wird.

Aufgrund geometrischer Anordnung und unterschiedlicher Dämpfung durch den Patienten unterschiedet sich der Empfangspegel des Pilottons in unterschiedlichen Antennenspulen und damit Empfangskanälen. Dabei ist die Antennenspule mit dem höchsten Pegel limitierend für einen maximalen Pegel des ausgesendeten Pilottons. Durch individuelle Dämpfungsglieder in einer Mehrzahl der oder in allen Empfangskanälen, insbesondere wenn diese solche Dämpfungswerte aufweisen, dass der Pegelunterschied des Pilottonsignals zwischen den Empfängern gegenüber den Empfangssignalen der Antennenspulen reduziert wird, kann das Pilottonsignal mit einem höheren Pegel ausgesendet werden und so das SNR des Pilottonsignals weiter erhöht werden.

In einer denkbaren Ausführungsform des erfindungsgemäßen Magnetresonanzsystems weist das Magnetresonanzsystem eine Steuerung auf, die ausgebildet ist, eine Dämpfung der frequenzabhängigen Dämpfungsglieder einzustellen. Beispielsweise kann ein einstellbarer Digital-Analog-Wandler als variabler Widerstand oder über eine ausgegebene Spannung eine variable Kapazität in Form einer PIN-Diode in einer Schaltung eines Dämpfungsgliedes variieren, wie sie nachfolgend zu den Figuren angegeben ist.

Dabei ist die Steuerung ausgebildet, die Dämpfungsglieder derart einzustellen, dass eine Signalpegeldifferenz zwischen Pilottonsignalen in Empfängern der Mehrzahl der Empfangskanäle reduziert wird. Dies kann beispielsweise erfolgen, indem die Steuerung ein Programm gemäß einem erfindungsgemäßen Verfahren zum Betrieb des Magnetresonanzsystems ausführt, das nachfolgend erläutert wird.

In dem erfindungsgemäßen Verfahren ermittelt die Steuerung in einem Schritt Signalpegel des Pilottonsignals in der Mehrzahl an Empfängern. Es ist beispielsweise denkbar, dass digitale Empfänger mittels einer Fouriertransformation das Pilottonsignal ausfiltern und dessen Pegel bestimmen. Denkbar sind aber auch andere Filter wie Bandpassfilter, die das Pilottonsignal separieren. Möglich wäre es aber auch, dass in Abwesenheit eines Magnetresonanzsignals und bei aktivem Pilotton ein Pegel am Empfänger im Rahmen eines Kalibriervorgangs ermittelt wird, wobei im Wesentlichen lediglich das Pilottonsignal erfasst wird und vorzugsweise auf aufwändige Filtermaßnahmen verzichtet werden kann.

In einem weiteren Schritt stellt die Steuerung eine Dämpfung der frequenzabhängigen Dämpfungsglieder derart ein, dass eine Signalpegeldifferenz zwischen Pilottonsignalen in Empfängern der Mehrzahl der Empfangskanäle reduziert wird. Mit anderen Worten, durch die Einstellung der Dämpfungsglieder werden die Pegel der Pilottonsignale in den Empfangskanälen angeglichen.

Dazu ist es beispielsweise denkbar, dass zunächst ein Empfangskanal ermittelt wird, der den niedrigsten Signalpegel für das Pilottonsignal aufweist. Für die anderen Empfangskanäle ermittelt die Steuerung die Pegeldifferenz zu diesem Empfangskanal. Anschließend stellt die Steuerung die jeweiligen Dämpfungsglieder der anderen Empfangskanäle so ein, dass deren Signalpegel reduziert wird, vorzugsweise im Wesentlichen auf das den Signalpegel des Empfangskanals mit dem niedrigsten Signalpegel. Liegt der Signalpegel eines Empfangskanals beispielsweise 6 dB über dem Signalpegel des schwächsten Empfangskanals, so kann die Dämpfung des Dämpfungsgliedes beispielsweise um 6 dB, aber auch um 7 oder 8 dB oder mehr, erhöht werden.

In einem weiteren Schritt wird ein Sendepegel des Pilottons so eingestellt, dass der Signalpegel der Empfangskanäle vor dem A/D-Wandler einen vorbestimmten Signalpegel nicht überschreitet. Der vorbestimmte Signalpegel kann dabei durch ein zu erwartenden Signalpegel für das Magnetresonanzsignal und/oder Eingangspegel am A/D-Wandler bestimmt sein.

Liegt beispielsweise der zu erwartende Signalpegel für das Magnetresonanzsignal bei 0 dBFS, also einem maximalen zu verarbeitenden Eingangspegel des A/D-Wandlers, und der Signalpegel des Pilottonsignals des Empfangskanals mit dem niedrigsten Signalpegel bei -18 dBFS, so werden z.B. die Dämpfungsglieder der anderen Empfangskanäle so von der Steuerung eingestellt, dass deren Signalpegel -18 dBFS nicht überschreitet. Anschließend kann der Signalpegel des Pilottons um 12 dB angehoben werden, wenn ein Pegelabstand von - 6 dB zwischen den maximalen zu erwartenden Magnetresonanzsignalen und dem Pilottonsignal erwünscht ist.

Die Pegelangaben im Beispiel sind dabei nur beispielhaft und können auch andere Werte annehmen. Auch ist es denkbar, dass beispielsweise der Empfangskanal mit dem höchsten Pegel für das Pilottonsignal ermittelt wird, die Dämpfung der Dämpfungsglieder in den anderen Empfangskanälen entsprechend reduziert wird, um einen im Wesentlichen gleichen Signalpegel für das Pilottonsignal zu erzielen, und anschließend des Sendepegel des Pilottons erniedrigt oder erhöht wird.

Auf vorteilhafte Weise können durch eine adaptive Anpassung der Dämpfungsglieder und des Pilottons, insbesondere für mehrere Empfangskanäle, ein zuverlässigeres Erkennen von Bewegungen des bzw. im Patienten sichergestellt werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanzsystems;
- Fig. 2: eine beispielhafte Ausführungsform eines Empfangspfades eines erfindungsgemäßen Magnetresonanzsystems;
- Fig. 3: eine beispielhafte Ausführungsform eines Dämpfungsgliedes für die erfindungsgemäße Magnetresonanzvorrichtung;
- Fig. 4: eine beispielhafte Ausführungsform eines Dämpfungsgliedes für die erfindungsgemäße Magnetresonanzvorrichtung.

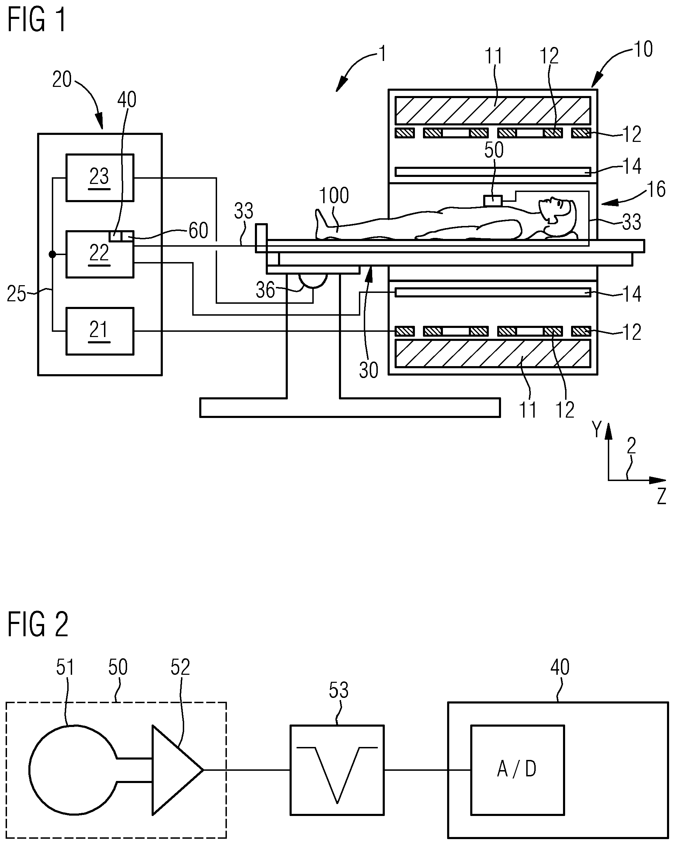

Fig. 1 zeigt eine schematische Darstellung einer Ausführungsform eines erfindungsgemäßen Magnetresonanzsystems 1.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. die Orientierung und den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungspulse können über die Körperspule 14 oder auch über eine lokale Sendeantenne in den Patienten 100 abgestrahlt werden.

Die Hochfrequenzeinheit 22 weist auch einen Empfänger 40 zum Empfang bzw. Aufbereitung eines Magnetresonanzsignals aus dem Patienten 100 auf, das von der Lokalspule 50 aufgenommen wird und über eine Signalverbindung zu dem Empfänger 40 überträgt. Der Empfänger 40 ist darüber hinaus auch ausgebildet, einen Pilotton zu empfangen und auszuwerten.

Die Hochfrequenzeinheit 22 kann auch einen Pilottonsender 60 aufweisen, der einen Pilotton an eine Pilotton-Sendeantenne, möglichweise auch in der Lokalspule 50, zum Auskoppeln über eine Signalverbindung überträgt. Denkbar ist aber auch ein separater Pilottonsender 60, der unabhängig vom Magnetresonanzsystem 1 agiert.

Eine Steuerung 23 kommuniziert über einen Signalbus 25 mit der Gradientensteuerung 21 und der Hochfrequenzeinheit 22.

Auf dem Patienten 100 ist eine Lokalspule 50 angeordnet, die über eine Anschlussleitung 33 mit der Hochfrequenzeinheit 22 und deren Empfänger verbunden ist.

Vorzugsweise weist die Lokalspule 50 weiterhin eine Antenne auf, mit der ein Pilotton in den Körper des Patienten 100 ausgesendet bzw. induziert werden kann.

Fig. 2 zeigt eine beispielhafte Ausführungsform eines Empfangspfades eines erfindungsgemäßen Magnetresonanzsystems 1.

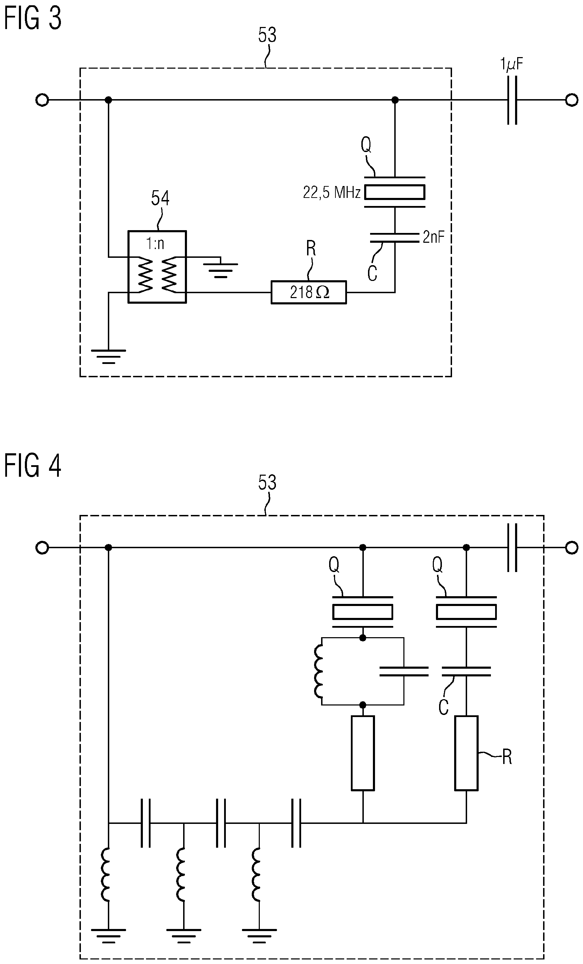

In der Lokalspule 50 nimmt eine Antennenspule 51 das Pilottonsignal und Magnetresonanzsignale aus dem Patienten 100 auf. Die empfangenen Signale werden von einem Low-Noise-Verstärker LNA 52 verstärkt. Anschließend dämpft ein Notch-Filter 53 selektiv das Pilottonsignal im Empfangspfad um einen vorbestimmten Wert. Details zu dem Notch-Filter 53 sind zu Fig. 3 angegeben.

Denkbar sind aber auch andere Formen des Dämpfungsgliedes. Beispielsweise kann ein schmalbandiges Notch-Filter auch durch ein Quarz-Filter realisiert werden, das vorteilhafter Weise eine hohe Frequenzstabilität aufweist.

Die Bandbreite eines Notch-Filters 53 bzw. eines schmalbandigen Bandpassfilters kann beispielsweise auch durch zwei gekoppelte resonante Filter wie z.B. Parallel- oder Serien-Schwingkreise beeinflusst und eingestellt werden. Denkbar ist die Realisierung des Dämpfungsgliedes auch durch ein Hoch- bzw. Tiefpass-Filters, dessen Flanke zwischen dem Magnetresonanzsignal und dem Pilottonsignal liegt und durch die Dämpfung das gewünschte Amplitudenverhältnis einstellt. Rauschanteile außerhalb der Frequenzbereiche von Magnetresonanzsignal und Pilottonsignal können dabei durch weitere Filter unterdrückt werden. Beispielsweise wirkt bereits die Antennenspule 51, die im Empfangsfall üblicherweise resonant auf das Magnetresonanzsignal abgestimmt ist, bereits als Bandpassfilter, der derartige Frequenzanteile außerhalb reduziert. Auch kann durch ein weiteres Bandpass- bzw. komplementäres Hochpass- oder Tiefpassfilter außerhalb liegende Frequenzanteile unterdrücken.

Anschließend werden in dem Empfänger 40 das Magnetresonanzsignal und das Pilottonsignal von einem A/D-Wandler digitalisiert und ausgewertet. Vorzugsweise werden das Pilottonsignal und das Magnetresonanzsignal in unterschiedliche digitale Signalströme aufgeteilt, die unterschiedlichen Verarbeitungsprozessen zugeführt werden. Dabei werden insbesondere Amplitudenschwankungen des Pilottonsignals auf charakteristische Veränderungen für z.B. Atembewegung und Herzschlag ausgewertet. Aus dem Magnetresonanzsignalen hingegen werden durch Bildrekonstruktion Bilddaten gewonnen. Dabei können dennoch Abhängigkeiten in den Prozessen auftreten, indem beispielsweise Magnetresonanzsignale nur weiterverarbeitet werden, wenn z.B. keine Bewegungen im Pilottonsignal oder wenn vorbestimmte Bewegungsphasen erkannt werden.

Die Verteilung der einzelnen Funktionsgruppen in Fig. 2 ist dabei nur für eine Ausführungsform beispielhaft. Denkbar sind gemäß der Erfindung auch andere Varianten. Beispielsweise könnte das Dämpfungsglied 53 auch integraler Teil der Lokalspule 50 oder des Empfängers 40 sein. Auch ist es möglich, die Reihenfolge im Empfangsweg zu verändern oder Funktionen zusammenzulegen, beispielsweise eine Filterfunktion in den LNA zu integrieren.

Fig. 3 zeigt eine beispielhafte Ausführungsform eines Dämpfungsgliedes für die erfindungsgemäße Magnetresonanzvorrichtung. Die Ausführungsform beruht auf einem Notch-Filter unter Verwendung eines Quarzes, hier mit einer Beispielfrequenz von 22,5 MHz. Das vorgeschlagene Dämpfungsglied hat eine Ein- und Ausgangsimpedanz von 50 Ohm, sodass es in übliche Empfänger-Schaltkreise in den Hochfrequenzpfad eingegliedert werden kann.

Ein einfaches Notch-Filter kann durch einen Quarz bereitgestellt werden, der an eine Signalleitung angeschlossen und in Serie mit einer Kapazität C und einem Widerstand R zu einer Signalmasse verbunden ist. Randbedingungen ist dabei die Impedanz der Signalleitung. Die maximale Dämpfung ist dabei über den Wert R des Serienwiderstandes wählbar, wobei die Mittenfrequenz und die Bandbreite des Notch-Filters dann durch die Eigenschaften des Quarzes sowie den Werten für C und R bei Einhaltung der Impedanz der Signalleitung vorgegeben ist.

In Fig. 3 ist eine Möglichkeit dargestellt, wie die Bandbreite eines Notch-Filters mit Quarz vergrößert wird.

Im Resonanzfall wird die Impedanz des Quarzes im Wesentlichen durch den internen Verlustwiderstand bestimmt. Mittels eines Transformators 54 mit einem Windungsverhältnis 1:n wird der Widerstand, bestehend aus der Serienschaltung des Widerstandes R und des quarzinternen Verlustwiderstandes, an der Signalleitung um den Faktor 1/(1+n)² reduziert. Dazu sind die Primärseite des Transformators und eine Serienschaltung aus Quarz Q, Kapazität C und Widerstand R und Sekundärseite des Transformators 54 antiparallel zwischen der Signalleitung und einer Signalmasse angeordnet. Das Windungsverhältnis der Windungen auf der Primärseite zu den Windungen auf der Sekundärseite ist dabei 1:n. Daher kann der Widerstand, bestehend aus der Serienschaltung des Widerstandes R und des quarzinternen Verlustwiderstandes, um den Faktor (1+n)² durch Vergrößern des Widerstandswertes für R um diesen Faktor erhöht werden, wobei die Impedanz an der Signalleitung insgesamt unverändert bleibt. Dadurch wird gleichzeitig die Bandbreite des Dämpfungsgliedes mit dem Transformator 54 um den Faktor (1+n)² vergrößert. Auf vorteilhafte Weise wird so ein zusätzlicher Freiheitsgrad beim Design des Dämpfungsgliedes gewonnen.

Fig. 4 zeigt eine weitere beispielhafte Ausführungsform eines Dämpfungsgliedes. Im Unterschied zu der Fig. 3 wird hier eine zusätzliche Verbreiterung der Bandreite erzielt, indem zwei Resonanzelemente unterschiedlicher Resonanzfrequenz, hier zwei Quarzresonatoren, miteinander gekoppelt werden. Die Kopplung ist hier durch eine Parallelschaltung realisiert, wobei der Kopplungsgrad unter anderem durch die Werte für die Widerstände R beeinflussbar ist. Da Quarze Q üblicherweise mit gewissen Standardfrequenzen verfügbar sind, wird eine unterschiedliche Resonanzfrequenz erreicht, indem den beiden Quarzen unterschiedliche reaktive Elemente in Serie geschaltet werden. So können mit zwei identischen Quarzen dennoch unterschiedliche Resonanzfrequenzen für die beiden Resonanzelemente erreicht werden.

Bei Magnetresonanzsystemen (1) mit einer Mehrzahl an Empfängern (40) für das Pilottonsignal ist es vorzugsweise vorgesehen, dass alle diese Empfänger (40) ein erfindungsgemäßes Dämpfungsglied aufweisen. Dies ist beispielsweise der Fall, wenn eine Lokalspule (50) eine Matrix an Antennenspulen aufweist. Durch Empfang des Pilottonsignals auf mehreren Kanälen kann die Zuverlässigkeit des Pilottons verbessert werden. Die Dämpfung der jeweiligen Dämpfungsglieder ist vorzugsweise so eingestellt, dass das Pilottonsignal nach dem Dämpfungsglied in den Empfängern (40) jeweils im Wesentlichen gleichen Pegel aufweist, sich also um weniger al 6 dB, 3 dB oder 1 dB unterscheidet.

Es ist auch denkbar, dass das bzw. die Dämpfungsglieder in ihrer Dämpfung von der Steuerung 23 einstellbar sind. Dies kann beispielsweise erreicht werden, indem die Kapazität C durch eine spannungsgesteuerte PIN-Diode und/oder der Widerstand R durch einen spannungsgesteuerten Widerstand ersetzt werden, die über einen D/A-Wandler angesteuert werden. Auch sind gesteuerte Umschalter zwischen Dämpfungsgliedern mit unterschiedlichen Dämpfungswerten in einem Empfangskanal möglich. Die Steuerung kann so die Pilottonvorrichtung an unterschiedliche Empfangsbedingungen durch unterschiedliche Anordnung und/oder Patienten anpassen. Mögliche Vorgehensweisen sind zu dem erfindungsgemäßen Verfahren näher erläutert.

Wenn nicht alle Empfänger (40) für die Auswertung des Pilottons genutzt werden sollen, ist es auch denkbar, dass Empfänger (40) ohne Pilotton-Funktion durch einen Bandpass nur das Magnetresonanzsignal durchlassen und das Pilottonsignal unterdrücken.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzsystem mit einer Pilottonvorrichtung, wobei das Magnetresonanzsystem (1) einen Empfänger (40) aufweist, der ausgebildet ist, ein Pilottonsignal und ein Magnetresonanzsignal gleichzeitig zu empfangen und auszuwerten, **dadurch gekennzeichnet, dass**
das Magnetresonanzsystem (1) ein frequenzabhängiges Dämpfungsglied aufweist, das ausgebildet ist, ein empfangenes Pilottonsignal relativ zu einem empfangenen Magnetresonanzsignal zu dämpfen.

2. Magnetresonanzsystem nach Anspruch 1, wobei das Dämpfungsglied ausgebildet ist, das Pilottonsignal gegenüber dem Magnetresonanzsignal um mehr als 3 dB, 6dB oder 12 dB zu dämpfen.

3. Magnetresonanzsystem nach Anspruch 2, wobei das Magnetresonanzsystem (1) ausgebildet ist, eine Dämpfung des Dämpfungsgliedes durch einen erhöhten Sendepegel der Pilottonvorrichtung für den Pilotton zu kompensieren.

4. Magnetresonanzsystem nach einem der vorhergehenden Ansprüche, wobei das Magnetresonanzsystem (1) einen Vorverstärker für das empfangene Pilottonsignal aufweist und das Dämpfungsglied als Teil des Vorverstärkers oder zwischen Vorverstärker und einem Analog-Digitalwandler in dem Empfänger angeordnet ist.

5. Magnetresonanzsystem nach einem der vorhergehenden Ansprüche, wobei das Dämpfungsglied einen Notch-Filter (53) aufweist.

6. Magnetresonanzsystem nach einem der vorhergehenden Ansprüche, wobei das Dämpfungsglied einen Quarz-Resonator aufweist.

7. Magnetresonanzsystem nach einem der vorhergehenden Ansprüche, wobei das Dämpfungsglied eine Mehrzahl an gekoppelten Resonatoren aufweist.

8. Magnetresonanzsystem nach einem der Ansprüche 5 bis 7, wobei das Dämpfungsglied einen Transformator aufweist, dessen Primärseite und Sekundärseite zwischen Signalpfad und Signalmasse antiparallel angeordnet sind, wobei ein Resonanzelement in Serie mit der Sekundärseite zwischen Signalpfad und Signalmasse angeordnet ist.

9. Magnetresonanzsystem nach einem der vorhergehenden Ansprüche, wobei das Magnetresonanzsystem eine Mehrzahl an Empfangskanälen aufweist, wobei die Mehrzahl an Empfangskanälen jeweils ein frequenzabhängiges Dämpfungsglied aufweist.

10. Magnetresonanzsystems nach Anspruch 9, wobei eine Dämpfung der frequenzabhängigen Dämpfungsglieder einstellbar ist und das Magnetresonanzsystem eine Steuerung (23) aufweist, die ausgebildet ist, eine Dämpfung der frequenzabhängigen Dämpfungsglieder derart einzustellen, dass eine Signalpegeldifferenz zwischen Pilottonsignalen in Empfängern der Mehrzahl der Empfangskanäle reduziert ist.

11. Verfahren zum Betrieb eine Magnetresonanzsystems nach Anspruch 10, wobei das Verfahren die Schritte aufweist:
Ermitteln von Signalpegeln des Pilottonsignals in der Mehrzahl an Empfängern;
Einstellen einer Dämpfung der frequenzabhängigen Dämpfungsglieder durch die Steuerung (23) derart, dass eine Signalpegeldifferenz zwischen Pilottonsignalen in Empfängern der Mehrzahl der Empfangskanäle reduziert wird;
Einstellen eines Signalpegels des Pilottons.

12. Computerprogrammprodukt mit Befehlen, die bei Ausführung durch ein Magnetresonanzsystem (1) nach Anspruch 10 das Magnetresonanzsystem (1) dazu veranlassen, ein Verfahren nach Anspruch 11 durchzuführen.
